# EUROPEAN PATENT SPECIFICATION

(11) **EP 0 653 221 B1**
(45) Date of publication and mention of the grant of the patent: **29.10.2008**
(21) Application number: 94203665.8
(22) Date of filing: 12.09.1991
(51) Int. Cl.: A61M 39/02, A61M 39/10

(54) **Needleless connector medical site**
Nadellose Injektionsstelle
Point de prise d'échantillon avec raccord sans aiguille

(30) Priority: 18.09.1990 US 584286
(43) Date of publication of application: 17.05.1995
(62) Divisional of application: 91920317.4
(73) Proprietor: Medex, Inc., Carlsbad, California 92008 (US)
(72) Inventor: Frank, Thomas P., Neenah, Wisconsin 54956 (US); Patzer, Charles R., Ashville, Ohio 43101 (US)
(74) Representative: Findlay, Alice Rosemary

(56) References cited:
- EP-A- 0 309 771
- EP-A- 0 343 953
- WO-A-89/06553
- DE-C- 3 303 718
- US-A- 5 006 114

## Description

This invention relates to medical sites, in particular to sample sites through which fluids are injected or withdrawn from a patient's circulatory system and, more particularly, to a sample site which does not require use of a needle for injecting or withdrawing samples and in which the access port is easily reached for disinfecting.

Sample sites for obtaining fluid access to a patient's circulatory system are well known. For example, a catheter inserted into a patient's blood vessel may be connected to a sample site such as shown in U.S. Patent No. 4,874,377. Alternatively, the sample site may be in-line with tubing connecting the catheter to a blood pressure monitoring transducer and/or a supply of saline or other solution, such as shown in U.S. Patent 5,148,811, entitled "Method and Apparatus for Sampling Blood", and which is a continuation-in-part of U.S. Patent No. 5,048,537.

Typical of such sites is that they include a housing having a liquid path extending therein and which is to be coupled to the patient's circulatory system to make the fluid connection such as through a catheter and/or the tubing of an intravenous system, for example, like the blood pressure monitoring apparatus shown in the aforementioned U.S. Patents 5,148,811 and 5,048,537. A sample site further typically includes an access port through which fluids may be introduced into the patient's circulatory system or from which blood may be withdrawn from the patient, both via the liquid path. By way of example, a needle may be received through a resilient stopper in the access port and on into the liquid path to couple a syringe or the like attached to the needle with the patient's circulatory system for injection/withdrawal of fluids. However, sampling sites requiring use of a needle present significant risk from needle sticks. As will be readily appreciated, needle sticks provide a mechanism for transfer of dangerous diseases, such as hepatitis or AIDS.

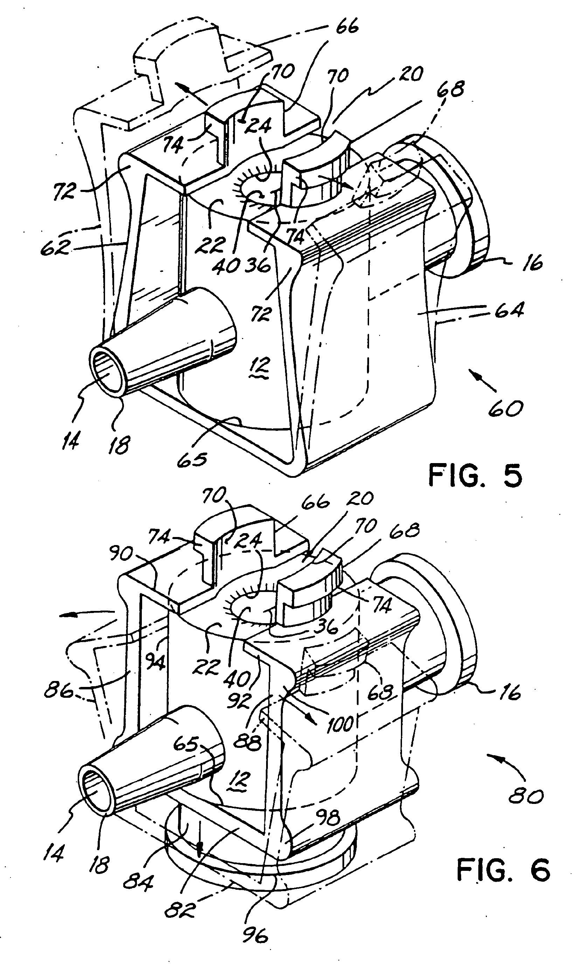

To reduce risk of needle sticks, sampling sites have been developed which eliminate needles. Such sampling sites provide an access port adapted for needleless connection with an external fluidic system such as a syringe or tubing. To this end, a cylindrical reservoir is provided on the sample site to provide a confined conduit between the needleless connector such as a male luer connector and a valve assembly within the sample site. One example is the stopcock side port shown in Figure 5 in the aforementioned U.S. Patent 5,148,811 in which the external fluidic system is connected thereto by securing the cuff of a male luer lock connector to the flanged top of a reservoir defined by the cylindrical wall of a female luer connector. Other needleless access ports are provided in which a blunt cannula, such as the tip or taper of a male luer connector, is receivable into the cylindrical reservoir. As shown, for example, in aforementioned U.S. Patent No. 4,874,377, the male luer connector tip impacts the valve assembly at the bottom of the female luer connector reservoir causing the valve to deflect and open under pressure of the tip of the male luer connector thereagainst. As the valve assembly opens, a channel for fluid communication between the liquid path within the sample site and the external fluidic system is created.

While such needleless sample sites reduce or eliminate needle stick problems, the cylindrical reservoir by which to secure the male luer connector to the sample site has introduced its own significant problems. In particular, the reservoir is difficult to clean and has prompted concern about contamination and the dangers of bacterial growth therein.

European Patent Application 0309771 describes a needleless sample site comprising a housing having a cylindrical aperture extending from an exterior wall through which a blunt cannula enters to make a connection to a liquid path. A seal is located in the aperture which is operable under pressure of the blunt cannula. A gap is provided between the aperture wall and the seal to accommodate expansion of the latter on opening thereof. When the site is not in use, a cover is securable over the housing exterior wall.

The invention provides a medical site for use with a medical male luer connector having a blunt male tip with a luer taper, as described in claim 1.

The present invention provides the benefits of a needleless sample site, while reducing the risk of contamination and bacterial growth. To this end, the reservoir above the valve assembly is eliminated and replaced with structure extending above the access port to provide the function of guiding a male luer connector taper against a seal member, but without a reservoir wall to obstruct ready access to the surface of the valve assembly for disinfecting. The structure may be arms which may be flanged to provide the function of locking a male luer lock connector thereto.

More specifically, the access port is defined by an apertured exterior wall of the sample site housing through which the taper of a male luer connector is to be received. The outer surface of the seal member which is contained within the housing normally blocks the aperture. The exterior wall adjacent the aperture and the outer surface of the seal member are easily wiped clean and, thus, are considered to be generally flush in the closed position of the seal member. Consequently, there is no reservoir defined about the aperture and seal surface from which contaminants or bacteria may be difficult to remove. The seal member is deflectable under pressure of the taper of a male luer connector passing into the aperture so as to open the seal member and provide fluid access to the liquid path in the sample site. When the male luer connector is withdrawn, the seal shuts to again block the aperture, but because there is no confined reservoir above the aperture, the exposed surface of the seal member and portions of the exterior wall adjacent the aperture (as well as the flanged arms) are readily accessible to a clinician for purposes of disinfecting the access port, such as by a wiping action between the flanged arms in a direction generally parallel the seal member and/or exterior wall surfaces. Consequently, there is less opportunity for contamination or bacterial growth with the sampling site of the present invention.

A female luer connector function is provided atop the access port but without the cylindrical reservoir normally provided thereby. To this end, structure, preferably in the form of a pair of arms, is provided adjacent the access port aperture such as to matingly receive a male luer connector taper therethrough while still providing generally unobstructed wiping access to the outer surface of the seal member and portions of the housing exterior wall adjacent the aperture. The arms may be flanged to lock the male luer connector thereto such that the arms may be seen as separate portions of a female luer connector and may thus be referred to as a split luer.

In one embodiment, the female luer connector portions or flanged arms are fixedly mounted to the housing exterior wall adjacent the aperture such that the inner or confronting surfaces of the flanged arms define an imaginary female luer connector cylinder about the aperture, but without the impediment of a complete cylindrical wall. In a further embodiment, the flanged arms are moveable from an operative position about the aperture to provide the female luer connector function to an inoperative position away from the sample site housing to provide completely unobstructed access to the outer surface of the seal member, as well as to the annular portion of the housing exterior wall completely surrounding the aperture. In accordance with this further embodiment, the flanged arms are mounted to the end of hinge arms so as to be moveable toward and away from the aperture. The hinge arms may be fixedly connected at their extreme ends to the housing and remote from the aperture and normally biased to urge the flanged arms away from the aperture. The hinge arms may then be compressed toward the sample site housing bringing the flanged arms adjacent the aperture for connecting to the male luer connector. Alternatively, the hinge arms may be slidably connected to the housing for sliding the flanged arms between the operative and inoperative positions. With the hinge arms connected to a frame member slidably disposed on a side of the housing opposite the access port, the hinge arms will ride along the side of the housing therebetween. In that event, the hinge arms are normally biased toward the housing to urge the flanged arms toward the aperture. As the frame member slides away from the access port, the hinge arms are pushed apart by the housing to separate the flanged arms and allow them to pass down over the side of the housing completely exposing the exterior wall and seal surface for disinfecting.

By virtue of the foregoing, there is thus provided a sample site with a flush-mounted needleless access port and a split or female luer structure including female luer connector portions so as to properly connect a male luer connector to the access port, but without obstructing wiping access to the seal member and surrounding regions of the housing for purposes of disinfection.

These and other objects and advantages of the present invention shall be made apparent from the accompanying drawings and description thereof.

The invention will now be described by way of example and with reference to the accompany drawings in which:
Fig. 1 is a perspective view of a first embodiment of a sample site in accordance with the principles of the present invention;
Fig. 2 is a top plan view of the sample site of Fig. 1;
Fig. 3 is a cross-sectional view of the sample site of Fig. 1 along line 3-3 of Fig. 2;
Fig. 4 is a cross-sectional view like that of Fig. 2, but with a syringe coupled thereto via a male luer connector;
Fig. 5 is a perspective view of a second embodiment of a sample site in accordance with the principles of the present invention;
Fig. 6 is a perspective view of a third embodiment of a sample site principles of the present invention; and
Fig. 7 is a diagrammatic view of an exemplary, closed blood sampling system incorporating a sample site of the present invention.

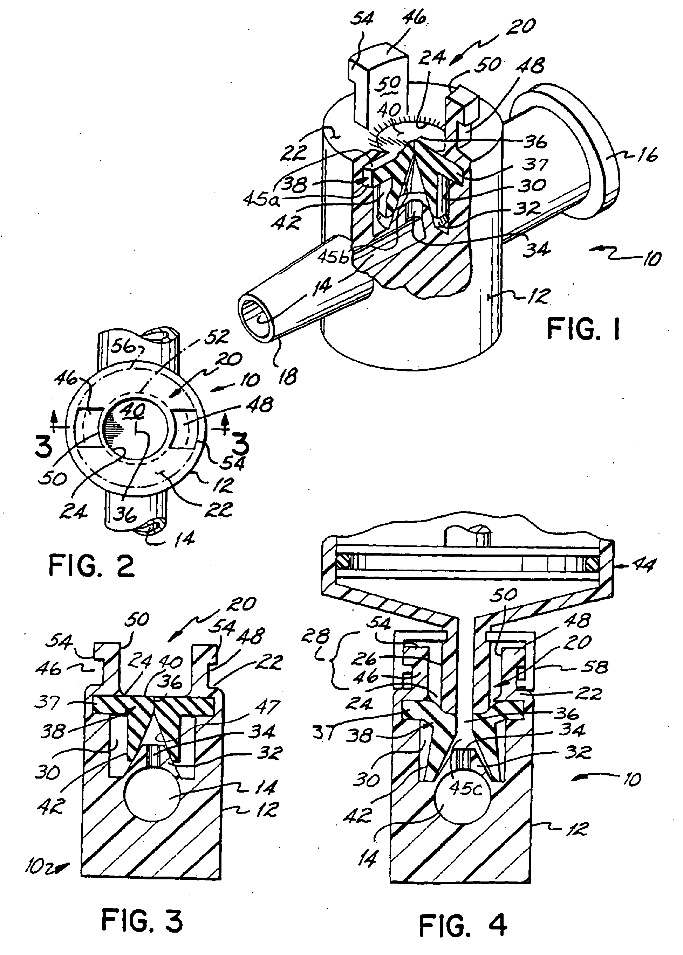

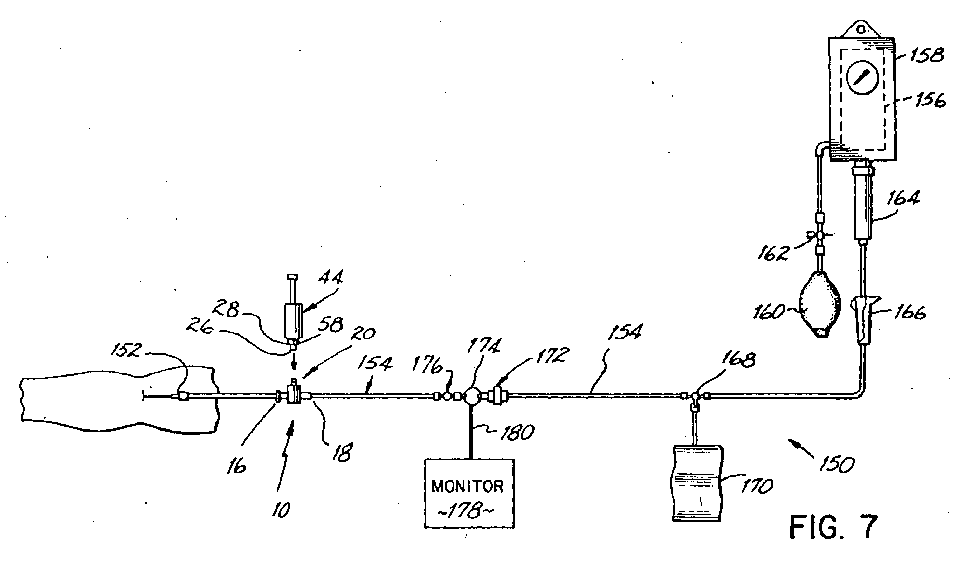

With reference to Figs. 1-4, there is shown one embodiment 10 of a sample site. Sample site 10 is defined by a cylindrical housing 12 having a liquid path 14 extending therein. At opposite ends of path 14 are female luer connector port 16 and male taper port 18 for connection with tubing or the like, such as for fluid communication with a patient's circulatory system (see Fig. 7). Housing 12 includes a needleless connector access port 20 between ports 16 and 18 defined along generally a planar, top exterior wall 22 of housing 12. Wall 22 includes an aperture 24 through which is to be received the tip or taper 26 of a male luer connector 28 for fluid communication with liquid path 14. Aperture 24 is sized to closely surround taper 26 when received therethrough.

A valve assembly is provided at aperture 24 and within recess 30 of housing 12 between top wall 22 and liquid path 14 by frustro-conical actuating member 32 fixedly positioned within housing 12 and a seal member 38. Actuating member 32 includes a lumen 34 therethrough transverse to and in fluid communication with liquid path 14. Lumen 34 is aimed at and sealed-off by normally closed channel 36 of diaphragm 37 of resilient seal member 38 positioned over actuator member 32. Seal member 38 includes tubular shaped hub 42 extending below seal upper surface 40 and with frustro-conical biassing portion 47 of hub 42 seated over actuating member 32. The top surface 40 of seal member 38 is generally flush top wall 22 when it is closed against or adjacent aperture 24. In this regard, the thickness of wall 22 adjacent aperture 24 is relatively thin in comparison to the length of the male luer connector tip 26 so as not to define a reservoir wall relative tip 26. Exterior wall 22 may be about 0.035-0.040 inches (0.9-1.0 mm) thick such that a wipe (not shown) passed across wall 22 will easily disinfect wall 22 and surface 40 without creating a well or reservoir for contaminants or bacteria to accumulate out of reach of the wipe as it passes across top wall 22. Alternatively, surface 40 may protrude from aperture 24. Thus, it will be appreciated that upper surface 40 of seal member 38 may be considered generally flush top wall 22 in the closed position inasmuch as the surface of the valve assembly is readily accessible to be wiped with disinfectant by wiping across top wall 22 from one side to the other.

As generally described in aforesaid U.S. Patent No. 4,874,377, under pressure from male lumen connector taper 26 against upper surface 40, seal member 38 deflects toward liquid path 14 and drives hub 42 against the conical surface of projection 32. As hub 42 rides over projection 32, channel 36 is caused to open revealing lumen 34 for fluid communication via tip 26 between liquid path 14 and an external fluidic system coupled to connector 28 such as a syringe 44 or tubing (not shown). Thus as can be seen in the figures, the outer wall 45A of seal member 38 is in contact with housing 12 and the inner wall 45B thereof defines a passage 45C for fluid to pass between lumen 34 and taper 26. When male luer connector 28 is removed, the resiliency of seal member 38 and biassing portion 47 urges channel 36 closed and also causes hub 42 to ride up frustro-conical actuating member 32 and reseal aperture 36 with outer surface 40 again adjacent aperture 24 and generally flush top wall 22.

To secure male luer connector 28 to sample site 10 while not otherwise obstructing wiping access to disinfect the exposed outer surface 40 of seal member 38 in aperture 24 and adjacent portions of external wall 22, a split luer arrangement is provided. To this end, extending upwardly and away from top wall 22 are a pair of flanged arms 46, 48. Arms 46, 48 are attached to top wall 22 at spaced apart locations adjacent aperture 24 such that the arcuate inner surfaces 50 of the arms define an imaginary cylinder 52 (shown in phantom line in Fig. 2) corresponding to the inner surface of a female luer connector cylinder. Similarly, flanges 54 of arms 46, 48 define an imaginary flange thread plane 56 (also shown in phantom line in Fig. 2) corresponding to the threading flange of a female luer connector onto which may be threadably received rotatable threaded cuff 58 of a male luer lock connector 28, as shown in Fig. 4. Arms 46, 48 thus define a pair of spaced apart female luer connector portions.

In the embodiment shown in Figs. 1-4, inner surface 50 and flange 54 of each arm 46, 48 define approximately fifteen percent (15%) of the circumference of a female luer connector cylinder and/or flange (a combined total of 30%) to thereby functionally provide the female luer connector functions of guiding tip 26 and locking connector 28 in place, respectively, while leaving access port 20 otherwise generally unobstructed. Thus, a clinician (not shown) may readily disinfect access port 20 and flange arms 46, 48 by wiping across the surface of top wall 22 such as in the direction from port 16 to port 18 with a disinfectant material such as with an alcohol wipe to disinfect the area about aperture 24. As a consequence, the needless sample site of the present invention eliminates the need to use needles with their attendant risks, while greatly reducing the risk of contamination and bacterial growth normally of great concern with prior needleless sample sites.

An alternative embodiment 60 of a sample site is shown in Fig. 5. Sample site 60 is substantially similar to sample site 10 with the exception of the provision of a modified split luer connector. In the sample site of embodiment 60, the entire surface of top wall 22 may be completely freed from obstruction by placing the split luer in an inoperative position. To this end, the split luer of embodiment 60 is provided by a pair of hinge arms 62, 64 connected such as by sonic welding or glue to bottom wall 65 of housing 12 and defining at their extremities a pair of female luer connector portions or flanged arms 66, 68. Hinge arms 62, 64 may be living hinges and are normally in an inoperative position shown in dashed line in Fig. 5 with arms 66, 68 outwardly of housing 12 so as to expose the entirety of top wall 22 and seal member outer surface 40 (as well as the inner walls 70 of arms 66, 68) to be easily and readily wiped clean for purposes of disinfection.

Hinge arms 62, 64 are thickened as at 72 adjacent flanged arms 66, 68 to provide a convenient place for a clinician (not shown) to grip hinge arms 62, 64 and compress them together to bring female luer connector portions 66, 68 into an operative position shown in solid line in Fig. 5 to provide the female luer connector function for receiving a male luer connector 28 as in the case of connector portions 46, 48 described in connection with sample site 10. When male luer connector 28 is removed, hinge arms 62, 64 urge the split luer apart into the inoperative position. The inner wall 70 and flange 74 of each flanged arm 66, 68 comprise about twenty-five percent (25%) of the circumference of a female luer connector cylinder and flange, or about fifty percent (50%) total.

A yet further alternative embodiment 80 is shown in Fig. 6. Sample site 80 is substantially similar to sample site 60 with the exception of the mechanism for moving female luer connector portions 66, 68 between the operative and inoperative positions shown in solid and dashed line, respectively. To this end, a frame member 82 is slidably supported on an annular extension 84 attached to bottom 65 of housing 12. Attached to frame member 82 are a pair of hinge arms 86, 88 with shelves, 90, 92 carrying flanged arms 66, 68 respectively. Hinge arms 86, 88 are normally biased toward side wall 94 of housing 12, such that with frame member 82 slid against bottom 65, shelves 90, 92 pass over housing top wall 22 to position flanged arms 66, 68 into the operative position adjacent aperture 24 to provide the female luer connector function. To disinfect the access port, frame member 82 is slid away from bottom 65 toward flange wall 96 of annular extension 84. As the frame member is thus moved, top wall 22 and shelves 90, 92 cooperate to urge hinge arms 86, 88 outwardly of housing 12 and away from aperture 24 allowing flanged arms 66, 68 to ride astride housing side wall 94 into the inoperative position completely exposing access port 20 for disinfecting such as by wiping a disinfectant material thereacross. Hinge arms 86, 88 are thickened top and bottom as at 98, 100 to facilitate sliding frame member 82 by finger pressure thereagainst in the direction desired to slide frame member 82. As shown in Fig. 6, hinge arms 86, 88 thus move in a direction parallel lumen 34 and transverse liquid path 14. The hinge arms could alternatively be mounted to slide parallel liquid path 14 and transverse lumen 34.

By virtue of the foregoing, there is provided a split luer arrangement to simulate a female luer connector, but without the reservoir which would otherwise impede ready access to access port 20 including surface 40 of seal member 38 and the adjacent annular portion of top wall 22 completely surrounding exposed surface 40 for disinfecting thereof, to reduce the likelihood that contamination of bacterial growth might occur. Although housing 12 has been shown as a T-connection, the access port and split luer combination of the present invention may be provided in other types of sample sites. Thus, the liquid path within housing 12 may be coaxial with lumen 34 rather than transverse thereto and may have only one of ports 16, 18 connected thereto. Similarly, ports 16 and 18 could form part of a Y-connector rather than a T-connector. Still further, the flush mounted needleless access port and split luer lock arrangement could also be provided at each of ports 16, 18.

Use of the sample site 10, 60 or 80 will be described in connection with a closed blood sampling system 150 as shown in Fig. 7. System 150 includes a catheter 152 for insertion into a patient's blood vessel and connected in a series via tubing 154 to a bag 156 of saline solution. Bag 156 is wrapped in a pressure cuff 158 which may be inflated by an inflation squeeze bulb 160 through a stopcock 162. The solution flows out of bag 156 through conventional drip chamber 164. A roller clamp 166 may be mounted on tubing 154 adjacent drip chamber 164 in order to selectively block the flow of solution from bag 156 toward the patient. Connected immediately downstream of roller clamp 166 is a shunted stopcock 168 coupled to a waste collection bag 170. Downstream of stopcock 168 is a flush device 172, pressure transducer 174, and three-way stopcock 176. Pressure transducer 174 is electrically connected to monitor 178 by cable 180 for monitoring the patient's blood pressure. A transducer of the type disclosed in U.S. Patent No. 4,920,972 may be employed. Operation of the above is described in greater detail in the aforementioned U.S. Patent 5,148,811.

In use, the sample site is situated between catheter 152 and three-way stopcock 176 by coupling port 16 to catheter 152 and port 18 to stopcock 176 to continue the in-line fluid path between bag 156 and catheter 152. Syringe 44 may be connected in fluid communication therewith by first wiping access port 20 with a disinfectant and then, if they are movable, placing the female luer connector portions of the sample site into the operative position. The male luer connector 28 is then fitted to access port 20 by inserting tip 26 thereof between the inner walls of the split luer and into the aperture to deflect seal member 38 and open the valve assembly under pressure of tip 26 to reveal lumen 34 and provide a fluid path between syringe 44 and liquid path 14 coupled to catheter 152. Since the male luer connector is of the locking type, the cuff 58 thereof may be rotated onto the flanges of the split luer to maintain the connection.

After injecting or sampling as desired, syringe 44 is removed by withdrawing male luer connector tip 26, allowing seal member 38 to reclose. The access port 20 will also be accessible (or made accessible by movement of the female luer connector portions to the inoperative position) to allow access port 20 to again be disinfected by wiping thereacross.

By virtue of the foregoing, there is thus provided a needleless connector sample site in which the access port is generally unobstructed to provide wiping access for disinfecting, while eliminating the risk of needle sticks common to needle-based sample sites and to improve sterility and reduce risk of infection otherwise feared from prior needleless connector sample sites.

It will be appreciated that the sample site may be utilized as an injection site when coupled to the venous side of a patient's circulatory system, as a sample port when connected to the arterial side, or as a link to connect an exterior fluidic system to an otherwise closed fluidic system without opening the closed system, thereby avoiding the possibility of reflux in the closed system. Reference to sample site will thus be appreciated as a reference to any of the above.

## Claims

1. A medical site (10,60,80) for use with a medical male luer connector having a blunt male tip (26) with a luer taper and a threaded locking cuff (58) about the male luer taper tip, the site (10,60,80) comprising a housing (12), a liquid path (14) extending into the housing, an apertured exterior wall (22) on the housing through which the male luer taper tip enters the housing (12) to make fluid connection to the liquid path, a seal element (38) retained by and within the housing (12), the seal element (38) having an outer surface (40) exposed adjacent the exterior wall aperture (24), and female luer structure (46,48,66,68) connected to the housing (12), **characterised in that** the female luer structure (46,48,66,68) comprises female luer locking connector structure (54,74) which extends in a radially outward direction with respect to the aperture (24), the female luer locking connector structure (54, 74) defining a thread plane onto which the threaded locking cuff (58) may be threaded to hold the male luer taper tip (26) within the aperture (24), and **in that** the female luer structure (46,48,66,68) is connected to the housing (12) and disposed with respect to the aperture (24) in a manner allowing aseptic wiping across the outer surface (40) of the seal element (38) and adjacent portions of the housing (12), the outer surface (40) of the seal element (38) being generally such with the exterior wall aperature (24).

2. A medical site as claimed in Claim 1 wherein the female luer structure (46,48,66,68) comprises female luer connector portions (46,48,66,68) formed on the housing.

3. A medical site as claimed in Claim 2 wherein the female luer connector portions (46,48,66,68) define 30% of a complete circumference of the thread plane.

4. A medical site as claimed in any one of Claims 2 and 3 wherein the female luer connector portions (46,48,66,68) are rigidly connected to the housing (12).

5. A medical site as claimed in any one of Claims 2 to 4 wherein the female luer connector portions (46,48,66,68) are connected for movement relative to the housing (12) between an operative position and an inoperative position.

6. A medical site as claimed in any one of Claims 2 to 5 wherein the female luer connector portions (46,48,66,68) are elevated relative to the aperture (24) and the exposed outer surface (40).

7. A medical site as claimed in any one of Claims 2 to 6 wherein the female luer connector portions are defined by a pair of oppositely disposed lugs (46,48,66,68).

8. A medical site as claimed in Claim 1 wherein the female luer structure is disposed below the aperture (24) and the exposed outer surface (40).

9. A medical site as claimed in Claim 8 wherein the female luer structure is defined by a pair of oppositely disposed lugs (66,68).

10. A medical site as claimed in any preceding Claim in which the seal member (38) includes a diaphragm (37) which presents the outer surface (40), the diaphragm (37) including a channel (36) which may be opened under pressure from the luer taper (26).

11. A medical site as claimed in Claim 10 further including a resilient biasing portion (47) behind the diaphragm (37) and extending toward the channel (36) to normally bias the channel closed.

12. A medical site as claimed in Claim 11 wherein the seal member (38) includes a tubular portion (42) connected to the diaphragm (37) and wherein the tubular portion (42) carries the biasing portion (47).

13. A medical site as claimed in any preceding Claim wherein the seal member (38) includes an outer wall (45A) in contact with the housing (12) and an inner wall (45B) defining a passage (45C) for receiving fluid within a path extending through the luer taper (26).

14. A medical site as claimed in any preceding Claim in which the outer surface (40) of the seal member (38) is flat.

15. A medical site as claimed in any preceding Claim in which the outer surface (40) of the seal member (38) protrudes from the aperture (24).

## Patentansprüche

1. Medizinische Stelle (10, 60, 80) für die Verwendung mit einem männlichen Luer-Anschluss im medizinischen Bereich mit einer stumpfen männlichen oberen Spitze (26) mit einem Luer-Kegel und einer Verschlussmanschette mit Gewindeelement (58) um die männliche Luer-Kegelspitze herum, wobei die Vorrichtung (10, 60, 80) ein Gehäuse (12), einen Flüssigkeitsdurchgang (14), der sich in das Gehäuse hinein erstreckt, eine Außenwand (22) mit Öffnung an dem Gehäuse, durch welche die männliche Luer-Kegelspitze in das Gehäuse (12) eintritt, um eine Verbindung für die Flüssigkeit mit dem Flüssigkeitsdurchgang herzustellen, ein Dichtungselement (38), welches durch die Gehäuse (12) und innerhalb des Gehäuses (12) gehalten ist, wobei das Dichtungselement (38) eine Außenfläche (40) aufweist, welche frei liegend an die Außenwandöffnung (24) angrenzt, sowie eine weibliche Luer-Struktur (46, 48, 66, 68), die mit dem Gehäuse (12) verbunden ist, umfasst, **dadurch gekennzeichnet, dass** die weibliche Luer-Struktur (46, 48, 66, 68) eine weibliche Luer-Lock-Anschlussstruktur (54, 74) umfasst, welche sich radial in Richtung nach außen in Bezug auf die Öffnung (24) erstreckt, wobei die weibliche Luer-Lock-Anschlussstruktur (54, 74) eine Gewindeebene definiert, auf welche die Verschlussmanschette mit Gewindeelement (58) angebracht werden kann, um die männliche Luer-Kegelspitze (26) innerhalb der Öffnung (24) zu halten und dass die weibliche Luer-Struktur (46, 48, 66, 68) mit dem Gehäuse (12) verbunden ist und unter Bezug auf die Öffnung (24) in einer Weise ausgeführt ist, welche aseptisches Wischen über die Außenfläche (40) des Dichtungselements (38) sowie über angrenzende Bereiche des Gehäuses (12) ermöglicht, wobei die Außenfläche (40) des Dichtungselements (38) im Allgemeinen mit der Außenwandöffnung (24) abschließt.

2. Medizinische Stelle nach Anspruch 1, wobei die weibliche Luer-Struktur (46, 48, 66, 68) weibliche Luer-Anschlussbereiche (46, 48, 66, 68) umfasst, welche an dem Gehäuse gebildet sind.

3. Medizinische Stelle nach Anspruch 2, wobei die weiblichen Luer-Anschlussbereiche (46, 48, 66, 68) 30 % eines kompletten Umfangs der Gewindeebene definieren.

4. Medizinische Stelle nach einem der Ansprüche 2 und 3, wobei die weiblichen Luer-Anschlussbereiche (46, 48, 66, 68) fest mit dem Gehäuse (12) verbunden sind.

5. Medizinische Stelle nach einem der Ansprüche 2 bis 4, wobei die weiblichen Luer-Anschlussbereiche (46, 48, 66, 68) so angebracht sind, dass sie in Bezug auf das Gehäuse (12) zwischen einer funktionsfähigen Position und einer nicht funktionsfähigen Position bewegt werden können.

6. Medizinische Stelle nach einem der Ansprüche 2 bis 5, wobei die weiblichen Luer-Anschlussbereiche (46, 48, 66, 68) in Bezug auf die Öffnung (24) und die frei liegende Außenfläche (40) erhöht sind.

7. Medizinische Stelle nach einem der Ansprüche 2 bis 6, wobei die weiblichen Luer-Anschlussbereiche durch ein Paar von gegenüberliegenden Vorsprüngen (46, 48, 66, 68) definiert sind.

8. Medizinische Stelle nach Anspruch 1, wobei sich die weibliche Luer-Struktur unterhalb der Öffnung (24) und der frei liegenden Außenfläche (40) befinden.

9. Medizinische Stelle nach Anspruch 8, wobei die weibliche Luer-Struktur durch ein Paar von gegenüberliegenden Vorsprüngen (66, 68) definiert ist.

10. Medizinische Stelle nach einem der vorhergehenden Ansprüche, wobei das Dichtungselement (38) ein Diaphragma (37) beinhaltet, welches die Außenfläche (40) darstellt, wobei das Diaphragma (37) einen Kanal (36) beinhaltet, welcher durch den Druck von dem Luer-Kegel (26) geöffnet werden kann.

11. Medizinische Stelle nach Anspruch 10, welche ferner einen elastischen Vorspannungsbereich (47) hinter dem Diaphragma (37) enthält, der sich in Richtung auf den Kanal (36) erstreckt, um den Kanal allgemein in eine geschlossene Position vorzuspannen.

12. Medizinische Stelle nach Anspruch 11, wobei das Dichtungselement (38) einen röhrenförmigen Bereich (42) beinhaltet, welcher mit dem Diaphragma (37) verbunden ist und wobei der röhrenförmige Bereich (42) den Vorspannungsbereich (47) trägt.

13. Medizinische Stelle nach einem der vorhergehenden Ansprüche, wobei das Dichtungselement (38) eine Außenwand (45A), die in Kontakt mit dem Gehäuse (12) steht, sowie eine Innenwand (45B) beinhaltet, welche eine Passage (45C) definieren, um Flüssigkeit innerhalb eines Durchgangs, welcher sich durch den Luer-Kegel (26) erstreckt aufzunehmen.

14. Medizinische Stelle nach einem der vorhergehenden Ansprüche, wobei die Außenfläche (40) des Dichtungselements (38) flach ist.

15. Medizinische Stelle nach einem der vorhergehenden Ansprüche, wobei die Außenfläche (40) des Dichtungselements (38) aus der Öffnung herausragt.

## Revendications

1. Un site médical (10, 60, 80) apte à être utilisé avec un connecteur Luer mâle médical comportant une pointe émoussée mâle (26) avec un effilement luer et un manchon de verrouillage fileté (58) autour de la pointe effilée luer mâle, le site (10, 60, 80) comprenant un boîtier (12), un chemin de circulation de liquide (14) se prolongeant dans le boîtier, une paroi extérieure ouverte (22) sur le boîtier à travers laquelle la pointe effilée luer mâle pénètre dans le boîtier (12) pour réaliser un raccord fluidique avec le chemin de circulation de liquide, un élément d'étanchéité (38) retenu par et contenu dans le boîtier (12), l'élément d'étanchéité (38) ayant une surface extérieure (40) exposée adjacente à l'ouverture (24) de la paroi extérieure et une structure luer femelle (46, 48, 66, 68) raccordée au boîtier (12), **caractérisé en ce que** la structure luer femelle (46, 48, 66, 68) comprend une structure de connecteur de verrouillage luer femelle (54, 74) qui se prolonge dans une direction radialement en sortie par rapport à l'ouverture (24), la structure du connecteur de verrouillage luer femelle (54, 74) définissant un plan de filetage sur lequel le manchon de verrouillage fileté (58) peut être fileté pour maintenir la pointe effilée luer mâle (26) dans l'ouverture (24) et **en ce que** la structure luer femelle (46, 48, 66, 68) est raccordée au boîtier (12) et disposée en regard avec l'ouverture (24) d'une manière permettant un balayage aseptique à travers la surface extérieure (40) de l'élément d'étanchéité (38) et des portions adjacentes du boîtier (12), la surface extérieure (40) de l'élément d'étanchéité (38) étant généralement au même niveau que l'ouverture (24) de la paroi extérieure.

2. Un site médical selon la revendication 1, dans lequel la structure luer femelle (46, 48, 66, 68) comprend des portions de connecteur luer femelle (46, 48, 66, 68) formées sur le boîtier.

3. Un site médical selon la revendication 2, dans lequel les portions du connecteur luer femelle (46, 48, 66, 68) définissent 30 % d'une circonférence complète du plan de filetage.

4. Un site médical selon l'une quelconque des revendications 2 et 3, dans lequel les portions du connecteur luer femelle (46, 48, 66, 68) sont rigidement raccordées au boîtier (12).

5. Un site médical selon l'une quelconque des revendications 2 à 4, dans lequel les portions du connecteur luer femelle (46, 48, 66, 68) sont raccordées en vue d'un mouvement par rapport au boîtier (12) entre une position opératoire et une position inopératoire.

6. Un site médical selon l'une quelconque des revendications 2 à 5, dans lequel les portions du connecteur luer femelle (46, 48, 66, 68) sont surélevées par rapport à l'ouverture (24) et à la surface extérieure exposée (40).

7. Un site médical selon l'une quelconque des revendications 2 à 6, dans lequel les portions du connecteur luer femelle sont définies par une paire de collerettes (46, 48, 66, 68) disposées l'une face à l'autre.

8. Un site médical selon la revendication 1, dans lequel la structure luer femelle est disposée en dessous de l'ouverture (24) et de la surface extérieure exposée (40).

9. Un site médical selon la revendication 8, dans lequel la structure luer femelle est définie par une paire de collerettes (66, 68) disposées l'une face à l'autre.

10. Un site médical selon une quelconque revendication précédente, dans lequel l'élément d'étanchéité (38) comprend une membrane (37) qui présente la surface extérieure (40), la membrane (37) ayant un conduit (36) qui peut être ouvert sous pression à partir de l'effilement luer (26).

11. Un site médical selon la revendication 10 comprenant en outre une portion de polarisation élastique (47) située derrière la membrane (37) et se prolongeant vers le conduit (36) pour polariser normalement le conduit fermé.

12. Un site médical selon la revendication 11, dans lequel l'élément d'étanchéité (38) comporte une portion tubulaire (42) connectée à la membrane (37) et dans lequel la portion tubulaire (42) transporte la portion de polarisation (47).

13. Un site médical selon une quelconque revendication précédente, dans lequel l'élément d'étanchéité (38) comprend une paroi extérieure (45A) en contact avec le boîtier (12) et une paroi intérieure (45B) définissant un passage (45C) pour la réception d'un fluide au sein d'un chemin se prolongeant dans l'effilement luer (26).

14. Un site médical selon une quelconque revendication précédente, dans lequel la surface extérieure (40) de l'élément d'étanchéité (38) est plate.

15. Un site médical selon une quelconque revendication précédente, dans lequel la surface extérieure (40) de l'élément d'étanchéité (38) dépasse de l'ouverture (24).
